**Europäisches Patentamt**

(19) **European Patent Office**

**Office européen des brevets**

(11) Publication number : **0 351 901 B1**

(12) **EUROPEAN PATENT SPECIFICATION**

(45) Date of publication of patent specification :
30.12.92 Bulletin 92/53

(51) Int. Cl.⁵ : **A61K 39/187,** C12N 5/02,
A61K 39/245

(21) Application number : **89201574.4**

(22) Date of filing : **15.06.89**

(54) **Swine kidney cell culture, and its use in vaccine production.**

(30) Priority : **23.06.88 NL 8801601**

(43) Date of publication of application :
**24.01.90 Bulletin 90/04**

(45) Publication of the grant of the patent :
**30.12.92 Bulletin 92/53**

(84) Designated Contracting States :
**AT BE CH DE ES FR GB GR IT LI LU NL SE**

(56) References cited :
**BE-A- 766 442**
**FR-A- 2 244 542**
**NL-A- 6 610 013**

(56) References cited :
**BIOLOGICAL ABSTRACTS, vol. 74, no. 5, 1982,**
**abstract no. 32153, Biological Abstracts Inc.,**
**Philadelphia, PA, US; M.H. JENSEN:"Hog**
**cholera antibodies in pigs vaccinated with an**
**Aujeszky-vaccine based on antigen produced**
**in IB-RS-2 cells", & ACTA VET.SCAND. 22(3/4):**
**517-523, 1981**
**BIOLOGICAL ABSTRACTS, vol. 73, no. 3, 1982,**
**abstract no. 18373, Biological Abstracts Inc.,**
**Philadelphia, PA, US; H. LAUDE etal.: "Hog**
**cholera diagnosis: Improved technique of**
**seroneutralization based on use of a cytolytic**
**virus strain in microplate"**

(73) Proprietor : **CENTRAAL DIERGENEESKUNDIG**
**INSTITUUT**
**Houtribweg 39**
**NL-8221 RA Lelystad (NL)**

(72) Inventor : **Terpstra, Catharinus**
**Boeier 02-94**
**NL-8242 CC Lelystad (NL)**

(74) Representative : **de Bruijn, Leendert C. et al**
**Nederlandsch Octrooibureau**
**Scheveningseweg 82 P.O. Box 29720**
**NL-2502 LS Den Haag (NL)**

## Description

The invention relates to a "swine kidney" cell culture which is suitable for the cultivation of viruses.

In various European, Asian and South American countries vaccines based on the so-called "Chinese" (C) strain of the hog cholera virus are currently used for combating hog cholera. The strain is a virus strain adapted on rabbits which, on the one hand, provides a high immunity in regard to the hog cholera virus and, on the other hand, presents virtually no danger to the health of the treated pigs. This strain was developed in Taiwan in the 1950s and 1960s (Lin, T.T.C. and Lee, R.C.T., 1981, An overall report on the development of a highly safe and potent lapinized hog cholera virus strain for hog cholera control in Taiwan; Nat. Sci. Council special publ. 5, 1-42) and reached Hungary via Vietnam and the People's Republic of China (Bognar, K. and Meszaros, J, 1963, Experiences with a lapinized hog cholera virus strain of decreased virulence; Act. Vet. Acad. Sci: Hung;, 13: 429-438). From there it spread to various Eastern and Western European countries.

The spleen and lymph nodes of rabbits infected with this C virus are used as the base material for the preparation of the vaccine based on the "Chinese" strain. One rabbit yields about 250-500 doses of freeze-dried product. At the time of the last hog cholera epidemic in the Netherlands (1983-1986) approximately 80% of all pigs in the Netherlands were vaccinated against hog cholera. On the basis of the number of slaughter animals, i.e. $19 \times 10^6$, the number of hog cholera vaccinations per year was $16.7 \times 10^6$. An additional 650,000 doses were used for the reinoculation of breeding sows. $17.3 \times 10^6 : 500 = 34,600$ rabbits were already needed solely for the production of the Dutch requirement of $17.3 \times 10^6$ doses of hog cholera vaccine per year.

Partly in view of the high consumption of rabbits, many attempts have been undertaken for the cultivation of the C virus, required for the preparation of the vaccine, in another host or in cell cultures, but these have not been successful because of the low yield or because of contamination of the primary cell cultures. For example, J. Biro et al. (Acta, Vet. Acad. Sci. Hung, 16, 293-300, 1966) initially appeared to have succeeded in adapting the virus of the Chinese strain of the hog cholera virus, which is marketed by the Hungarian Institute "Phylaxia" as a vaccine on the basis of production in rabbits under the name "Suvac", to sheep. However, after 30 passages this adapted strain lost its pathogenicity for rabbits and its immunizing power in pigs (see Table II on page 296) and nothing more has been heard of it since then.

In Rec. Med. Vet. 147, 937-953, 1971 the adaptation of the Chinese strain of the hog cholera virus to primary lamb kidney cells is described, the seventeenth passage being used as seed lot. The characteristics of this CL strain, which is marketed by IFFA-Merieux under the name "Pestiffa", have regularly been the subject of publications in Rev. Med. Vet. However, it was found that one lot of vaccine based on this CL strain was contaminated with the bovine virus diarrhoea virus, which was the cause of the birth of dead and sick piglets and the slaughter of the animals in some breeding stations; Wensvoort, G. and Terpstra, C. Bovine viral diarrhoea infections in piglets born to sows vaccinated against swine fever with contaminated vaccine (Res. Vet. Science, in press).

Furthermore, Kasza, L. et al. has developed a "swine kidney" (SK-6) cell line from the kidneys of a six-month-old slaughter pig: ("Establishment, viral susceptibility and biological characteristics of a swine kidney cell line SK-6"; Res. Vet. Sci:, 13: 1972, 46-51). This cell line was cultivated as a monolayer (SK6-M) on a solid medium. The ROVAC virus reproduced in this cell system without a visible cytopathological effect (CPE) being detected. Studies by the Applicant have shown that the virus of the C virus strain attenuated in rabbits also reproduced in SK6-M cultures, likewise without a CPE occurring. However, the virus yield remained low, as can be seen from the growth curve of the sixth virus passage on SK6-M (Table A). The adaptation of C virus to SK6-M was therefore not continued further.

TABLE A  Growth after 6 passages of C virus on SK6-M cells.

| Date | Hours after infection | Log plaque-forming units/ml | |
| --- | --- | --- | --- |
| | | Supernatant | Cell pellet |
| 23.04.85 | 24 | 1.5 | <1.3 |
| 24.04.85 | 48 | <1.3 | <1.3 |
| 25.04.85 | 72 | 1.8 | <1.3 |
| 26.04.86 | 96 | 2.8 | <1.3 |

As the monolayer system for the cultivation of viruses harbours many disadvantages and as the use of a large number of test animals to prepare vaccines is becoming increasingly socially less acceptable, the Applicant has attempted to adapt the cells of the abovementioned SK6-M culture in such a way that these can grow in a suspension culture.

After prolonged experimental studies and after many failures, the Applicant has surprisingly succeeded in adapting the swine kidney cells known from the prior art (SK6 cells) in such a way that these can grow in suspension. More particularly, the adaptation of the cell line from monolayer culture to suspension culture has been carried out with the aid of an enriched Eagles minimum essential medium using various "passages". The composition of the enriched Eagles minimum essential medium used for this purpose was as follows:

The weights and volumes of the ingredients are based on the preparation of 100 l.

| | | |
| --- | --- | --- |
| 1. | L-Arginine HCL, FBZ[a]); art. 1543 | 3.82 g |
| 2. | L-Glutamine; Ajinomoto | 26.55 g |
| 3. | L-Histidine HCl $H_2O$ FBZ; art. 4350 | 2.13 g |
| 4. | L-Isoleucine FBZ; art. 5362 | 4.82 g |
| 5. | L-Leucine FBZ; art. 5360 | 4.82 g |
| 6. | L-Lysine HCl, FBZ; art. 5700 | 6.64 g |
| 7. | L-Phenylalanine, FBZ; art. 7256 | 3.00 g |
| 8. | L-Methionine, FBZ; art. 5707 | 1.37 g |
| 9. | L-Threonine, FBZ; art. 8411 | 4.37 g |
| 10. | L-Tryptophan, FBZ; art. 8374 | 0.73 g |

| | | |
|---|---|---|
| 11. | L-Tyrosine, FBZ; art. 8371. | 3.28 g |
| | Dissolved separately at 70°C | |
| 12. | L-Valine, FBZ; art. 8495 | 4.28 g |
| 13. | Inositol Merck, art. 4731 NF X 11 | 0.32 g |
| 14. | D (+) Ca Pantothenate, FBZ; art. 2316 | 0.20 g |
| 15. | Thiamine HCl, Ph VIII[b]) | 0.20 g |
| 16. | Folic acid, ICN, cat. no. 101725 | 0.18 g |
| 17. | Pyridoxal. HCL, FBZ, art. 7523 | 0.23 g |
| 18. | Nicotinamide, FBZ; art. 6818 | 0.18 g |
| 19. | Choline chloride DAB 8; Merck art. 500117 | 0.21 g |
| 20. | Riboflavine, FBZ; art. 7609. | 0.02 g |
| | Separately dissolved in 1M NaOH[c]) and added. | |
| 21. | L-Cysteine FBZ; art. 2837 | 2.18 g |
| 22. | NaCl, Ph VIII | 594.00 g |
| 23. | KCl, p.a. | 36.35 g |
| 24. | $CaCl_2.2H_2O$, p.a. | 24.15 g |
| 25. | $MgSO_4.7H_2O$, p.a. | 18.20 g |
| 26. | $NaH_2PO_4.2H_2O$, p.a. | 12.75 g |
| 27. | $Fe (NO_3).9H_2O$, p.a. | 9.1 ml |
| | Dissolved separately as 0.1% and then added to 1-26 | |
| 28. | Phenol Red Merck, art. 7241 | 1.35 g |
| 29. | $NaHCO_3$, p.a. | 250.00 g |
| 30. | D (+) glucose. $1H_2O$ for parenteral use Ph Eur. | 450.00 g |
| 31. | Pepton, Oxoid, code L37 | 200.00 g |
| 32. | LAH, ICN cat. nr. 102131 | 150.00 g |
| 33. | Tryptose phosphate Antibiotics | 150.00 g |
| 34. | Sodium benzyl penicillin Ph VIIl | 10.00 g |
| 35. | Streptomycin sulphate Ph VIII | 10.00 g |
| 36. | Neomycin sulphate Ph VIII | 10.00 g |

---

Ingredients 1-21 are dissolved in approximately two thirds of the final volume. 22-25, 26-29 and then 30-36 are then added successively.

(a) FBZ: "Für biochemische Zwecke", (For biochemical use) manufacturer Merck Darmstadt;

(b) Ph VlII meets the requirements specified in the Dutch Pharmacopoeia, edition VIII

(c) NaOH purum, EKA Ab Sweden.

While cultivating in suspension the starting material was trypsinized SK6-M cells of an "unknown" passage level. Although there is said to be a continuous culture in the case of suspension systems, one passage signifies one growth cycle of the culture, analogously to monolayer cultures. After one growth cycle the cells were drawn off and subjected to a subsequent growth cycle in a refreshed growth medium. After 20 "passages" a culture with a cell concentration of $1.5 \times 10^6$ cells/ml was obtained.

The invention therefore relates to SK6 cells which can grow in suspension and which are deposited with the Pasteur Institute under No. i-768. Advantages of the SK6 suspension culture (SK6-S) over the SK6 monolayer culture which may be mentioned are the significantly higher yield per unit volume and also the simple and controllable procedure.

The SK6-S culture according to the invention can be applied as growth substrate for many viruses, after adaptation as necessary. Examples of such viruses which may be mentioned are the "Chinese" strain of the hog cholera virus and possibly the Aujeszky virus.

More particularly, the abovementioned disadvantage of an inadequate virus yield in the case of a C vaccine strain adapted to rabbits in a SK6-M cell culture can be eliminated by using a cell culture of the SK6-S cell line in which a "Chinese" strain of the hog cholera virus adapted to this persists, as deposited with the Pasteur Institute under i-767.

It is considered highly possible that the C virus adapted to SK6-S cells can also be reproduced in numerous other mammalian cells, thereby obtaining high virus yields, in contrast to what is found with the starting virus. The invention therefore also relates to the cultivation of the SK6-S adapted C virus according to the invention in monolayer and suspension culture systems of all mammalian cells. It has been found that in contrast to the C virus adapted to rabbits and used as the starting virus, the C virus according to the invention, which is adapted to SK6-S, can reproduce outstandingly well in SK6-M cells.

This increase and the fact that the virus can be titrated on PK15 cells provide the proof that the C-SK6-S virus can reproduce in PK15 cells. The starting virus originating from rabbits, on the other hand, does not reproduce in PK15 cells. The characteristic that C-SK6-S virus can be cultured on PK15 cells can therefore be regarded as a "marker" for the adapted vaccine virus according to the invention.

With regard to the characterization of the C-SK6-S "seed lot" virus according to the invention, investigations were carried out to determine to what extent the "seed lot" virus has changed in relation to the starting or "mother" virus produced in rabbits. For this purpose a suspension of $4 \times 10^7$ PK15 cells in 20 ml of growth medium was infected simultaneously with $2 \times 10^5$ $TCID_{50}$ of the seed lot virus (MOI = 1:200). After the cells had grown to a monolayer in three days, this layer was frozen (-20°C) together with the supernatant medium and thawed, after which the suspension was titrated on PK15 cells. The virus titre of the suspension was $10^{5.1}$ $TCID_{50}$/ml. The total yield was therefore $20 \times 10^{5.1} = 2 \times 10^{6.1}$ $TCID_{50}$,

i.e. an increase of $\dfrac{2 \times 10^{6.1}}{2 \times 10^5}$ or a factor of 12.5

The C virus according to the invention, which is adapted to SK6-S, can be differentiated from the C virus produced in rabbits on the basis of the abovementioned differences. In principle, there are two routes available for this which will be investigated in more detail by the Applicant:

1) Differentiation of the two viruses at protein level.

A panel of 12 monoclonal antibodies (MCA's) has been prepared against the C-SK6 "seed lot" virus. The Applicant will, on the basis of further research, investigate whether these MCA's make the desired differentiation between the two viruses possible.

2) Differentiation of the two viruses at genome level.

The Applicant has succeeded in cloning the genome of the virulent Brescia strain of hog cholera virus over its entire length in the form of overlapping cDNA fragments. The RNA of this virus strain will probably be sequenced by the end of 1988. By means of cross-hybridization of C virus RNA produced in rabbits and the cDNA clones on the one hand and of C virus RNA produced in SK6-S cells and cDNA clones on the other hand, it is considered possible to detect differences in the nucleotide sequences of the two viruses.

Adaptation of the "Chinese" strain of the hog cholera virus to the SK6-S cell line and the vaccine preparation thereof.

For the adaptation of this C virus to the abovementioned SK6-S cell line which has been deposited with the Pasteur Institute, a 50 ml culture with a concentration of $5 \times 10^5$ SK6-S cells/ml was infected with 2 ml of a 10% rabbit spleen suspension. The titre of the spleen suspension was $10^5$ rabbit $ID_{50}$/ml. After infection, samples were taken from the culture at set times. The ratio of live to dead cells was determined by means of a vital colouring and the cells were examined for the presence of hog cholera antigen by means of immunofluorescence. From the start of the infection there was an increased mortality of the cells, but by timely pelleting the surviving cells by means of centrifuging and providing them with fresh medium it was possible to maintain the culture. After 15 suspension days, during which the culture had been provided six times (= 6 "passages") with fresh medium, the cell mortality decreased. At the same time the proportion of the SK6-S cells which was shown to be infected with C virus on the basis of an immunofluorescence test (IFT), increased from < 1% to > 50% (Table B).

## TABLE B

### Adaptation of C virus to SK6 suspension culture

| Date | No. | IFT | Remarks |
|---|---|---|---|
| | Passage | | |
| 25.03.85 | 1 | ± (CPE) | Day 2 a few pos. cells |
| 28.03.85 | 2 | ± (CPE) | Very large number of dead cells |
| 29.03.85 | 3 | ± (CPE) | A few pos. cells |
| 01.04.85 | 4 | ± | A few pos. cells |
| 03.04.85 | 4 | ± | A few pos. cells |
| 04.04.85 | 5 | + | Somewhat more cells |
| 09.04.85 | 6 | ++ | More pos. cells against neg. |
| 10.04.85 | 7 | ++ | More pos. cells against neg. |
| 11.04.85 | 8 | ++ | Somewhat fewer than pass. 6, an occasional pos. cell |
| 12.04.85 | 8 | ± | Cells brought to 300,000/ml |
| 15.04.85 | 9 | +++ | |
| 16.04.85 | 9 | +++ | |
| 17.04.85 | 9 | +++ | |
| 19.04.85 | 9 | +++ | |
| 22.04.85 | 10 | ++++ | |
| 23.04.85 | 11 | ++++ | 75% of the infected cells |
| 24.04.85 | 11 | +++ | Cell growth somewhat less |
| 26.04.85 | | ++++ | Fluorescence weaker |
| 01.05.85 | 12 | ++++ | Weak fluorescence but fairly full |
| 03.05.85 | 13-1 | ++++ | 80% of the cells definitely infected |
| 03.05.85 | 13-2 | ++++ | |
| 06.05.85 | 14-1 | ++++ | Fairly full |
| 06.05.85 | 14-2 | ++++ | Fairly full |
| 10.05.85 | 15 large | ++++ | |
| 10.05.85 | 15 small | ++++ | |
| 13.05.85 | 16 | 1+; 2-; 3±; | |
| | 1 to 5 | 4+++ and 5++ | |
| 17.05.85 | 17 | | Large and small lot |

```
-    : no positive cells/cup

±    : < 1 positive cell/cup

+    : > 1 positive cell/cup but < 1 positive cell/field of vision

++   : 2-10 positive cells/field of vision

+++  : 11-100 positive cells/field of vision

++++: > 100 positive cells/field of vision
```

Titrations, carried out on SK6-M cells in the microtitre system, however, showed no detectable virus titre for the first 10 "passages", probably because the C virus was still insufficiently adapted to enable it to reproduce in SK6-M cells. The virus was demonstrated in this system in "passage" 11. A growth curve of the virus was determined from this "passage". After 24 hours a titre of $10^{1.7}$ 50% tissue culture infective doses ($TCID_{50}$) per ml was measured. After 48, 72, 96 and 120 hours the virus titre was $10^{4.1}$, $10^{5.4}$, $10^{5.5}$ and $10^{4.9}$ $TCID_{50}$/ml respectively.

After 17 "passages" had been reached, a new series was started with the virus of "passage" 11, in which series not only the cell concentration and the percentage of IFT positive cells but also the virus titre was carefully followed (Table C). In this case a CPE occurred again with a ratio of live:dead cells of less than 1, as a consequence. The CPE phenomenon occurred the same number of times as addition of C virus, whether or not having been cultivated on SK6-S, to uninfected SK6-S cells. From "passage" 14 the percentage of IFT positive cells increased rapidly and a stable (CPE-resistant) cell population was selected. This cell population (C-SK6-S) was persistently infected with C virus and continuously produces cell-free virus. Despite the persisting infection, the C-SK6-S cell has the ability to reproduce in suspension.

Table C

Summary of passage of C virus on SK6-suspension culture

| Passage | Days after persistence | Cell concentration x $10^6$/ml | | % of cells IFT-positive | Titre $\log_{10}$/ml | Remarks |
|---|---|---|---|---|---|---|
| | | live | dead | | | |
| 11 | 3 | 0.78 | | | | $10^{5.5}$TCID$_{50}$ in 50 ml |
| 12 | 1 | 0.36 | 0.55 | <1 | 2.8 | |
| 13 | 2 | 0.44 | 0.84 | <1 | 2.8 | |
| 14 | 3 | 0.65 | 0.85 | 5-10 | | |
| | 2 | 0.58 | 0.78 | 85 | 5.3 | |
| | 3 | 0.70 | 0.53 | | | 50% fresh medium |
| 15 | 4 | 0.83 | 0.31 | 85 | 5.0 | |
| 16 | 3 | 0.98 | 0.31 | 15 | 3.7 | |
| 17 | 4 | 1.12 | 0.50 | 20 | 4.6 | |
| 18 | 3 | 1.29 | 0.43 | 80 | 4.9 | |
| 19 | 4 | 1.13 | | 65 | 4.5 | |
| 20 | 3 | 0.74 | 0.30 | 75 | 5.0 | |
| 21 | 3 | 1.14 | | 80 | 5.0 | |
| 22 | 4 | 1.66 | | 80 | 4.9 | |
| 23 | 4 | 0.63 | | 75 | 4.9 | |
| 24 | 3 | 0.19 | 0.65 | 75 | 4.9 | |
| 25 | 4 | 0.64 | | 70 | 5.1 | |
| 26 | 3 | 1.03 | 0.27 | 45 | 4.8 | |
| 27 | 4 | 0.95 | 0.40 | 80 | 5.6 | |
| 28 | 3 | 1.52 | | 90 | 4.7 | |
| 29 | 4 | 1.16 | | 90 | 4.5 | |
| 30 | 3 | 1.23 | | | 5.3 | |

From "passage" 14 the virus titre likewise increased abruptly to about $10^5$TCID$_{50}$/ml cell-free medium (Table C). The persisting infected C-SK6-S culture persisted to "passage" 30. The virus titre did not increase further during these passages.

To determine the safety margin of the adapted virus, the production from "passage" 30 of the C-SK6-S cell line was checked for the presence of virus contaminants and for effectiveness in SPF pigs. The product of this passage was found to be apathogenic in the inoculated pigs. It induced no antibodies against other pathogenic pig viruses, such as bovine virus, diarrhoea virus, foot-and-mouth virus types A, O and C, porcine parvovirus, transmissible gastroenteritis virus, swine vesicular disease and the virus of Aujeszky's disease, but did protect the animals against a challenge with virulent hog cholera virus.

Normally, a vaccine virus is frozen separately as "seed lot virus" after adaptation to the cell system. For the preparation of a vaccine lot, the cell system must subsequently first be cultivated, after which the virus must adapt again. An advantage of freezing the virus/cell combination according to the invention is the maximum preservation of the characteristics of the symbiotic virus/cell system. A low "passage" level of the virus/cell combination, on the one hand, has the advantage that the biological characteristics of the C-SK6-S virus are changed as little as possible with respect to the starting material (rabbit C virus) and, on the other hand, has the advantage that the safety margin with respect to "passage" 30 is as large as possible.

Therefore, after the effectiveness and inocuity of the virus produced by "passage" 30 had been established, a further SK6-S culture was infected with the virus from "passage 11". For this purpose $10^{6.4} TCID_{50}$ of the 72 h sample of "passage" 11 were added to a 300 ml culture of SK6-S. The course of the culture is summarized in Table D.

## TABLE D

### Preparation of "master cell seed" and "seed lot virus" of C-SK6-S

| Passage | | Days after persistence | Vol. litres | Cell concentration x $10^6$/ml | | % cells IFT-positive | Titre $\log_{10}$/ml | Remarks |
|---------|---|---------|-------------|----------------|---|----------|-------|---------|
| date | No | tence | | live | dead | positive | ml | |
| 27.01.86 | 11 | | 0.3 | 0.2 | | | | |
| 31.01.86 | 12 | 4 | 0.3 | 0.9 | 0.14 | 65 | | |
| 03.02.86 | 13 | 3 | 0.3 | 0.78 | - | 35 | | |
| 07.02.86 | 14 | 4 | 2x0.3 | 1.40 | - | 50 | | |
| 10.02.86 | 15 | 3 | 3 | 0.43 | - | 75 | | |
| 13.02.86 | 16 | 3 | 3 | 0.81 | - | 90 | | Channel out |
| 17.02.86 | 17 | 4 | 8 | | | | | |
| 21.02.86 | 18 | 4 | 45 | | | | 5.6 | |

19 Ampoules containing about $6 \times 10^7$ C-SK6-S cells in 10% DMSO (dimethyl sulphoxide) from "passage" 18 were frozen in liquid $N_2$. One ampoule of this "master cell seed" is intended for starting a new production series. In addition about 40 l of cell-free growth medium from "passage" 18 was stored as "seed lot" virus in liquid $N_2$. This lot, which has a virus titre of $10^{5.6} TCID_{50}$/ml, can be used as seed virus for a possible new passage series and/or for the preparation of end product lots. This C-SK6-S "seed lot" virus is deposited with the Pasteur Institute under No. i-767.

The following may be mentioned as quality characteristics of the C-SK6-S "seed lot" virus:

### 1) Harmless to pigs

- The "seed lot" virus is bacteriologically, mycologically and mycoplasmologically sterile.
- The "seed lot" virus has been checked for virological purity with respect to African hog cholera virus, bovine virus, diarrhoea virus, foot-and-mouth virus types A, O and C, porcine enterovirus serotypes 1 and 2, porcine parvovirus, transmissible gastroenteritis virus, swine vesicular disease virus and the virus of Aujeszky's disease by means of a repeated intramuscular administration to sensitive pigs. After investigation, the "seed lot" virus was found to have induced no antibodies against the said viruses.
- The "seed lot" virus does not cause any disease symptoms or other undesired side effects such as retarded growth and leukopenia in pigs susceptible to hog cholera, even when the pigs were treated with corticosteroids.
- Dissemination of the "seed lot" virus from inoculated pigs to non-inoculated contact animals was not established.
- After inoculation of sows during the first 35 days of pregnancy with an amount of "seed lot" virus equivalent to 400 doses of vaccine no transuterine transmission of vaccine virus was found.

2) Stability and storage life after freeze-drying

- After adding 5% lactose to the "seed lot" virus, the latter could be freeze-dried without a measurable loss of contagiousness. The freeze-dried product has a storage life of at least 12 months at 4°C and -20°C.

3) Effectiveness

- After 7 days pigs vaccinated with "seed lot" virus are resistant to an intramuscular infection with $10^{4.6}$ 50% lethal doses ($LD_{50}$) of a virulent hog cholera virus strain.
- The immunity lasts for at least 6 months.

## Claims

1. Cells of the "swine kidney" SK6 cell line as deposited with the Pasteur Institute under No. i-768.

2. Cell of the "swine kidney" SK6 cell line according to Claim 1, in which a "Chinese" strain of the hog cholera virus adapted thereto persists, which virus is deposited with the Pasteur Institute under No. i-767.

3. Method for cultivating hog cholera virus with the aid of a cell line, characterized in that the "swine kidney" SK6 cell line according to Claim 1 or 2 is applied in this method.

4. Hog cholera vaccine derived from the product obtained according to Claim 3.

5. "Chinese" strain of the hog cholera virus adapted to the SK6 cell line according to Claim 1 or 2, as deposited with the Pasteur Institute under No. i-767.

6. Method for culturing the "Chinese" strain of the hog cholera virus defined in Claim 5, using culture systems based on mammalian cells.

7. Vaccine derived from the product obtained using the method described in Claim 6.

## Patentansprüche

1. Zellen der "Schweinenieren" SK6-Zell-Linie, wie unter der Nr. i-768 beim Pasteur-Institut hinterlegt.

2. Zelle der "Schweinenieren" SK6-Zell-Linie nach Anspruch 1, in der ein an sie angepaßter "chinesischer" Stamm des Schweine-Cholera-Virus fortbesteht, der unter der Nr. i-767 beim Pasteur-Institut hinterlegt ist.

3. Verfahren zum Kultivieren von Schweine-Cholera-Virus mit Hilfe einer Zell-Linie,
   **dadurch gekennzeichnet,**
   daß in diesem Verfahren die "Schweinenieren" SK6-Zell-Linie nach Anspruch 1 oder 2 angewendet wird.

4. Schweine-Cholera-Impfstoff, von dem nach Anspruch 3 erhaltenen Produkt stammend.

5. "Chinesischer" Stamm des an die SK6-Zell-Linie nach Anspruch 1 oder 2 angepaßten Schweine-Cholera-Virus, wie unter der Nr. i-767 beim Pasteur-Institut hinterlegt.

6. Verfahren zum Züchten des in Anspruch 5 definierten "chinesischen" Stammes des Schweine-Cholera-Virus unter Verwendung von auf Säugetierzellen basierenden Kultursystemen.

7. Impfstoff, von dem unter Verwendung des in Anspruch 6 beschriebenen Verfahrens erhaltenen Produkt stammend.

**Revendications**

1. Cellules de la lignée cellulaire de "rein de porc" SK6, déposée à l'Institut Pasteur avec le numéro i-768.

2. Cellule de la lignée cellulaire de "rein de porc" SK6, conforme à la revendication 1, dans laquelle persiste une souche "Chinoise" du virus du choléra des porcs qui y est adaptée, lequel virus est déposé à l'Institut Pasteur avec le numéro i-767.

3. Procédé de culture du virus du choléra des porcs à l'aide d'une lignée cellulaire, caractérisé en ce que l'on utilise, dans ce procédé, une lignée cellulaire de "rein de porc" SK6, conforme à la revendication 1 ou 2.

4. Vaccin contre le choléra des porcs, dérivé du produit obtenu conformément à la revendication 3.

5. Souche "Chinoise" du virus du choléra des porcs adaptée à la lignée cellulaire SK6 conforme à la revendication 1 ou 2, déposée à l'Institut Pasteur avec le numéro i-767.

6. Procédé de culture de la souche "Chinoise" du virus du choléra des porcs, définie dans la revendication 5, à l'aide de systèmes de culture à base de cellules de mammifères.

7. Vaccin dérivé du produit obtenu selon le procédé décrit dans la revendication 6.